(19) Europäisches Patentamt
European Patent Office
Office européen des brevets

(11) Veröffentlichungsnummer: **0 273 173 B1**

(12) # EUROPÄISCHE PATENTSCHRIFT

(45) Veröffentlichungstag der Patentschrift: **03.07.91**

(51) Int. Cl.[5]: **C07C 255/03**, C07C 253/22, C07C 31/22

(21) Anmeldenummer: **87116921.5**

(22) Anmeldetag: **17.11.87**

(54) **Verfahren zur Herstellung von Fettsäurenitrilen und Glycerin aus Glyceriden.**

(30) Priorität: **21.11.86 DE 3639857**

(43) Veröffentlichungstag der Anmeldung:
**06.07.88 Patentblatt 88/27**

(45) Bekanntmachung des Hinweises auf die Patenterteilung:
**03.07.91 Patentblatt 91/27**

(84) Benannte Vertragsstaaten:
**AT BE CH DE ES FR GB IT LI NL SE**

(56) Entgegenhaltungen:
**EP-A- 0 000 916**

(73) Patentinhaber: **HOECHST AKTIENGESELLSCHAFT**
**Postfach 80 03 20**
**W-6230 Frankfurt am Main 80(DE)**

(72) Erfinder: **Stühler, Herbert, Dr.**
**Hochfellnstrasse 12**
**W-8269 Burgkirchen(DE)**
Erfinder: **Fischer, Kurt**
**Fischervorstadt 43**
**W-8265 Neuötting(DE)**

**EP 0 273 173 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Verfahren zur gleichzeitigen Herstellung von Fettsäurenitrilen und Glycerin aus Glyceriden, bei dem das Glycerid in einem Reaktionsgefäß bei einer Temperatur von 220 bis 300 °C mit Ammoniak in einer Menge von mindestens 200 Liter pro Kilogramm Glycerid pro Stunde in Gegenwart von für diese Umsetzung ausgewählten Katalysatoren umgesetzt und das bei der Glyceridumsetzung (oder Hauptreaktion) gebildete Produktgemisch aus dem Reaktionsgefäß ausgetragen wird, bis in dem ausgetragenen Produktgemisch, das im wesentlichen aus Wasser, Glycerin und Fettsäure und Fettsäureamid enthaltendem Fettsäurenitril besteht, praktisch kein Glycerin mehr enthalten ist (Ende der Glyceridumsetzung oder der Hauptreaktion), und bei dem anschließend (das heißt nach Beendigung der Glyceridumsetzung oder Glycerinaustragung) das Fettsäure und Fettsäureamid enthaltende Fettsäurenitril in das Reaktionsgefäß zurückgebracht und in Gegenwart des obengenannten Katalysators bei einer Temperatur von 240 bis 320 °C mit Ammoniak in einer Menge von 5 bis 150 Liter pro Kilogramm im Reaktionsgefäß insgesamt vorliegendem Fettsäure und Fettsäureamid enthaltendem Fettsäurenitril pro Stunde weiter umgesetzt wird (Nachreaktion) bis die gesamte Fettsäure und das gesamte Fettsäureamid in Fettsäurenitril umgewandelt sind (Ende der Nachreaktion).

Ein solches Verfahren ist aus der US-Patentschrift 4 234 509 (die der europäischen Patentschrift 0 000 916 äquivalent ist) bekannt. Dabei wird das ausgetragene, Fettsäure und Fettsäureamid enthaltende Fettsäurenitril nach Beendigung der Glycerid-Ammoniak-Reaktion (nach dem Ende der Hauptreaktion) in das Reaktionsgefäß gegeben, in dem dann unter den angegebenen Bedingungen in einer als Nachreaktion bezeichneten Umsetzung die Umwandlung der gesamten vorliegenden Fettsäure und des gesamten vorliegenden Fettsäureamids in Fettsäurenitril abläuft (vgl. US-Patentschrift 4 234 509, insbesondere Spalte 7, ab Zeile 54, und Anspruch 8). Bei diesem Verfahren wird also in einer 1. Stufe das eingesetzte Glycerid so lange mit Ammoniak umgesetzt, bis praktisch das gesamte zu erwartende Glycerin ausgetragen ist und darauf diese Umsetzung abgebrochen. Nun erst wird das bis zu diesem Zeitpunkt in einer gegebenenfalls auf 60 bis 120 °C beheizten Vorlage als 2. Phase (neben der Glycerin-Phase) angesammelte Fettsäurenitril mit einem mehr oder weniger großen Gehalt an Fettsäure und Fettsäureamid (die beide unerwünscht und deshalb in Fettsäurenitril umzuwandeln sind) in das Reaktionsgefäß gegeben (zu dem dort noch befindlichen Produktrest, der im wesentlichen ebenfalls aus Fettsäure und Fettsäureamid enthaltendem Fettsäurenitril besteht) und der Inhalt des Reaktionsgefäßes, das ist das gesamte (verunreinigte) Roh-Fettsäurenitril, in Gegenwart des obengenannten Katalysators bei einer Temperatur von 240 bis 320 °C mit Ammoniak in einer Menge von 5 bis 150 l pro kg Roh-Fettsäurenitril pro Stunde umgesetzt, bis Fettsäure und Fettsäureamid in Fettsäurenitril umgewandelt sind (bei dieser Umsetzung wird praktisch nur noch Reaktionswasser und der überschüssige Ammoniak aus dem Reaktionsgefäß ausgetragen).

Es hat sich herausgestellt, daß das in der US-Patentschrift 4 234 509 beschriebene Verfahren zur Herstellung von Fettsäurenitril und Glycerin aus Glyceriden einige Nachteile aufweist. So muß durch den zeitlich von der Hauptreaktion getrennten Beginn der Nachreaktion eine relativ lange Chargenzeit in Kauf genommen werden. Besonders die Qualität des Glycerins läßt zu wünschen übrig. Es enthält nicht nur eine beträchtliche Menge an Wasser, es ist auch oft mit Fettsäure, Fettsäureamid und Fettsäurenitril verunreinigt. Alle Beispiele der US-PS 4 234 509 (auch das Beispiel 16, in dem das eingangs geschilderte bekannte Verfahren näher erläutert ist) zeigen, daß zur Aufarbeitung des in der Vorlage angesammelten Glycerins und Roh-Fettsäurenitrils eine umfangreiche Wasserwäsche erforderlich ist. Es ist insbesondere erforderlich, das Roh-Fettsäurenitril vor dem Einbringen in das Reaktionsgefäß nach dem Ende der Hauptreaktion mit Wasser zu waschen, weil es neben Fettsäure und Fettsäureamid auch noch Glycerin enthält, das vor dem genannten Einbringen entfernt werden muß, weil es andernfalls im Fettsäurenitril verbleibt und weil es sich bei den relativ hohen Temperaturen der Nachreaktion zumindest teilweise zersetzen würde (Farb- und Geruchsbildung).

In der US-PS 4 234 509 ist neben der oben geschilderten Verfahrensweise zur Herstellung von Fettsäurenitril und Glycerin aus Glyceriden als weitere Variante eine solche beschrieben, bei der das ausgetragene Produktgemisch vor der Phasentrennung einer Fraktionierung unterworfen wird und die dabei von Fettsäurenitril und Glycerin abgetrennten Fettsäure und Fettsäureamid kontinuierlich in die Reaktion zurückgeführt werden (vgl. US-Patentschrift, Kol. 7, ab Zeile 23, und Anspruch 7). Es hat sich herausgestellt, daß diese 2. Verfahrensweise noch nachteiliger ist als die 1., zumal das erhaltene Fettsäurenitril auch noch farblich und geruchlich nicht voll befriedigt.

Schließlich werden für die in Rede stehende Umsetzung in der DE-OS 32 44 752 bestimmte Diorganozinnsulfonate als Katalysatoren empfohlen. Damit werden zwar bessere Ausbeuten an Glycerin und Fettsäurenitril erreicht, bezüglich der Reinheit der beiden Produkte, insbesondere bezüglich der Reinheit des Glycerins, gelten jedoch auch hier die oben erwähnten Nachteile.

Die Aufgabe der Erfindung besteht demnach darin, das eingangs genannte Verfahren dahingehend zu verbessern, daß die angeführten Nachteile weitgehend ausgeschaltet oder nicht mehr vorhanden sind. Dies wird erfindungsgemäß überraschend dadurch erreicht, daß das ausgetragene Fettsäure und Fettsäureamid enthaltende Fettsäurenitril anstelle der Rückführung nach der Glyceridumsetzung während der Glyceridumsetzung in die Reaktion zurückgeführt wird, wobei die oben angegebene Temperatur von 240 bis 320 °C und die oben angegebene Ammoniak-Menge von 5 bis 150 l (das sind die Bedingungen der Nachreaktion) dann eingestellt werden, sobald die Glyceridumsetzung praktisch beendet ist.

Beim erfindungsgemäßen Verfahren wird also Fettsäure und Fettsäureamid enthaltendes Fettsäurenitril (auch Roh-Fettsäurenitril genannt) während der Glyceridumsetzung in die Reaktion zurückgebracht. Im Gegensatz dazu wird beim Stand der Technik das Roh-Fettsäurenitril erst nach dem Ende der Glyceridumsetzung weiterbehandelt, oder es wird nicht Roh-Fettsäurenitril, sondern nur die daraus durch Fraktionierung abgetrennte Fettsäure und das abgetrennte Fettsäureamid in die Reaktion zurückgeführt.

Es war überraschend, daß mit der erfindungsgemäßen Verfahrensweise ein derart positiver Effekt erreicht wird, daß die oben angegebenen Nachteile der bekannten Verfahren praktisch ausgeschaltet werden. Ein wesentlicher Grund für den unerwartet hohen Effekt dürfte darin liegen, daß durch die erfindungsgemäße Rückführung des Roh-Fettsäurenitrils, insbesondere durch die darin enthaltenen Fettsäurenitrile, ein sogenannter Schleppeffekt geschaffen wird, durch den eine eindeutige, das heißt stetige und rasche Austragung des Glycerins erreicht wird.

Das erfindungsgemäße Zurückführen von Roh-Fettsäurenitril wird vorzugsweise kontinuierlich oder stufenweise durchgeführt. Das kontinuierliche Zurückführen wird vorzugsweise so gestaltet, daß die während der Glyceridumsetzung in der Zeiteinheit ausgetragene Menge an Roh-Fettsäurenitril im wesentlichen in der gleichen Zeiteinheit und im wesentlichen in der gleichen Menge zurückgebracht wird. Das stufenweise Zurückführen wird vorzugsweise so gestaltet, daß in festgelegten Zeitintervallen jeweils eine bestimmte Menge an Roh-Fettsäurenitril zurückgebracht wird. Von den beiden Varianten ist das kontinuierliche Zurückführen bevorzugt. Das Zurückbringen von Roh-Fettsäurenitril wird in der Regel nicht unmittelbar nach dem Beginn des Austragens aufgenommen werden, da es von dem gleichzeitig damit ausgetragenen Glycerin und Wasser klarerweise erst abzutrennen ist. Nach einer bevorzugten Ausführungsform des erfindungsgemäßen Verfahrens wird, ebenso wie beim bekannten Verfahren, das bei der Glyceridumsetzung gebildete Produktgemisch über eine Kondensationseinrichtung in eine beheizte Vorlage ausgetragen. Das erfindungsgemäß bevorzugte Verfahren, bei dem das Produktgemisch aus dem Reaktionsgefäß über eine mit einer Kondensationseinrichtung verbundenen Leitung ausgetragen wird und bei dem die in der Kondensationseinrichtung kondensierten Produktkomponenten Glycerin und Fettsäure und Fettsäureamid enthaltendes Fettsäurenitril in einer beheizten Vorlage in eine untere Glycerin-Phase und in eine obere, Fettsäure und Fettsäureamid enthaltende Fettsäurenitril-Phase (Roh-Fettsäurenitril) abgeschieden werden, ist dadurch gekennzeichnet, daß die Verbindungsleitung vom Reaktionsgefäß zur Kondensationseinrichtung während der Glyceridumsetzung im wesentlichen auf die Temperatur erhitzt wird, die der Reaktionstemperatur entspricht, und daß das in der Vorlage abgeschiedene Fettsäure und Fettsäureamid enthaltende Fettsäurenitril während der Glyceridumsetzung in die Reaktion zurückgeführt wird. Auch bei dieser Ausführungsform des erfindungsgemäßen Verfahrens wird das Zurückbringen von Roh-Fettsäurenitril vorzugsweise kontinuierlich oder stufenweise durchgeführt, wobei das kontinuierliche Zurückbringen bevorzugt ist. Dies wird in einfacher Weise dadurch erreicht, daß Roh-Fettsäurenitril aus der Vorlage kontinuierlich in das Reaktionsgefäß zurückfließt über eine Leitung, die den Kopf der Vorlage mit dem Kopf des Reaktionsgefäßes verbindet. Das Zurückfließen wird erst nach Auffüllung der Vorlage mit Glycerin als untere Phase und Roh-Fettsäurenitril als obere Phase beginnen. Das dadurch entstehende, mehr oder weniger kurze Zeitintervall zwischen dem Beginn des Austragens und dem Beginn des Rückführens von Roh-Fettsäurenitril ist im Vergleich zur gesamten Reaktionszeit der Glyceridumsetzung vernachlässigbar. Das Zurückführen von Roh-Fettsäurenitril in die Reaktion wird zweckmäßigerweise spätestens dann aufgenommen, wenn bereits bis zu 20 Gew.-%, vorzugsweise bis zu 15 Gew.-%, von dem insgesamt zu erwartenden Glycerin/Roh-Fettsäurenitril-Gemisch ausgetragen sind. Es wird auch nicht das gesamte ausgetragene Roh-Fettsäurenitril zurückgeführt, weil je nach Größe der Vorlage zwangsläufig ein Rest an Roh-Fettsäurenitril zurückbleibt. Dieser Rest ist im Vergleich zum gesamten Roh-Fettsäurenitril sehr gering. Bei der beschriebenen Art des Zurückführens von Roh-Fettsäurenitril in die Glyceridumsetzung wird also die während der Umsetzung in der Zeiteinheit ausgetragene Menge an Roh-Fettsäurenitril im wesentlichen in der gleichen Zeiteinheit und im wesentlichen in der gleichen Menge zurückgebracht. Neben dem Zurückführen von Roh-Fettsäurenitril in die Glyceridumsetzung wird erfindungsgemäß die Leitung, durch die das Produktgemisch in die Kondensationseinrichtung gelangt, erhitzt, und zwar auf jene Temperatur, die im wesentlichen gleich der Reaktionstemperatur während der Glyceridumsetzung ist. Es wird im wesentlichen das gesamte Leitungsstück zwischen dem Kopf des Reaktionsgefäßes und der

Kondensationseinrichtung auf der in Rede stehenden Temperatur gehalten. Das Erhitzen erfolgt zweckmäßigerweise mit Hilfe einer elektrischen Heizung, zum Beispiel mit einem elektrischen Heizmantel, der um die Leitung gelegt ist, oder mit Hilfe einer Heizflüssigkeit, die in dem Doppelmantel, mit dem die Leitung versehen ist, zirkuliert. Die Kondensationseinrichtung, die beispielsweise aus einem Kühler besteht, in dessen unteren Teil die Verbindungsleitung vom Reaktionsgefäß einmündet, ist so gesteuert, daß die genannten Komponenten Glycerin und Roh-Fettsäurenitril im wesentlichen vollständig kondensieren, während das Reaktionswasser und der überschüssige Ammoniak gasförmig austreten.

Erfindungsgemäß ist es bevorzugt, die Vorlage und damit ihren Inhalt, das ist die Glycerin-Phase und die Rohnitril-Phase, auf eine Temperatur von 60 bis 200 °C, vorzugsweise 140 bis 175 °C, zu erhitzen und bei dieser Temperatur zu halten, bis die Glyceridumsetzung (die Hauptreaktion) praktisch beendet ist. Die Vorlage kann auch noch während der Nachreaktion bei der erfindungsgemäß einzustellenden Temperatur gehalten werden. Das Erhitzen der Vorlage erfolgt zweckmäßigerweise mit Hilfe einer elektrischen Heizung, zum Beispiel mit einem elektrischen Heizmantel, der um die Vorlage gelegt ist, oder mit Hilfe einer Heizflüssigkeit, die in dem Doppelmantel, mit dem die Vorlage versehen ist, zirkuliert. Mit dem erfindungsgemäßen Erhitzen der Vorlage wird erreicht, daß die beiden Phasen praktisch frei sind von Wasser und Ammoniak, und daß sie darüber hinaus voneinander vollständig getrennt sind, das bedeutet, die Glycerin-Phase enthält praktisch kein Rohnitril und die Rohnitril-Phase enthält praktisch kein Glycerin. Daraus resultiert, daß das aus der Vorlage als untere Phase abgenommene Glycerin bereits ein hochwertiges Glycerin ist. Der durch das erfindungsgemäße Erhitzen der Vorlage erreichte Effekt kann noch erhöht werden, wenn die Glycerin-Phase leicht gerührt wird.

Bei der bevorzugten Ausführungsform des erfindungsgemäßen Verfahrens wird demnach im einzelnen vorzugsweise so vorgegangen, daß man die Verbindungsleitung zwischen Reaktionsgefäß und Kondensationseinrichtung und die Vorlage auf die erfindungsgemäß einzustellenden Temperaturen erhitzt, das mit Beginn der Glyceridumsetzung aus der Kondensationseinrichtung in die Vorlage gelangende, praktisch wasser-und ammoniakfreie Produktgemisch in eine untere Glycerin-Phase und in eine obere Roh-Fettsäurenitril-Phase abscheiden läßt und nach Auffüllung der Vorlage jene Menge an Roh-Fettsäurenitril über die Leitung von der Vorlage zum Reaktionsgefäß einfach zurückfließen läßt, die mit dem Roh-Fettsäurenitril/Glycerin-Gemisch kontinuierlich in die Vorlage gelangt ist. Nach dem Ende der Hauptreaktion wird der Inhalt des Reaktionsgefäßes (das ist das gesamte Roh-Fettsäurenitril) unter den Bedingungen der Nachreaktion behandelt. Bei dieser Verfahrensweise wird das angestrebte Glycerin als untere Phase aus der Vorlage und das angestrebte Fettsäurenitril aus dem Reaktionsgefäß nach Beendigung der Nachreaktion abgezogen. Das Ablassen des Glycerins aus der Vorlage kann kontinuierlich erfolgen, und zwar im wesentlich in der Menge, in der es in die Vorlage gelangt und sich dort als untere Phase absetzt.

Mit dem erfindungsgemäßen Verfahren wird gleichzeitig und direkt ein besonders reines Glycerin und ein besonders reines Fettsäurenitril in hoher Ausbeute erhalten. Die beiden Produkte brauchen deshalb keinen umständlichen und zeitraubenden Reinigungsoperationen, wie Entgasung und Wasserwäsche, unterworfen werden. Das erhaltene Glycerin stellt praktisch ein hochwertiges Glycerin dar. Das gleichzeitig erhaltene Fettsäurenitril enthält zwar noch den eingesetzten Katalysator, es ist jedoch praktisch frei von den besonders unerwünschten Verunreinigungen Fettsäure und Fettsäureamid. Der Katalysator kann leicht, beispielsweise destillativ, entfernt werden. Beim erfindungsgemäßen Verfahren wird ferner die Glycerid-Umsetzung in einer kürzeren Zeit erreicht als bei den bekannten Verfahrensvarianten. Dies resultiert offensichtlich aus der neuen Rückführtechnik. Die Gesamtreaktionszeit wird auch noch dadurch verkürzt, daß kein Zeitverlust zwischen Hauptreaktion und Nachreaktion vorkommt. Das erfindungsgemäße Verfahren weist also eine kürzere Chargenzeit auf als die bekannten Verfahrensvarianten. Es ist im Vergleich zu diesen noch einfacher und führt in besonders wirtschaftlicher Weise zu reinen Produkten in hoher Ausbeute.

Bezüglich der an sich bekannten Maßnahmen des erfindungsgemäßen Verfahrens wird auf die eingangs genannten Druckschriften US-Patentschrift 4 234 509 und EP-Patentschrift 0 000 916 hingewiesen, in denen diese Maßnahmen und ebenso die in Betracht kommenden Glyceride (die Ausgangsstoffe) ausführlich beschrieben sind.

Auch beim erfindungsgemäßen Verfahren sind die Ausgangsstoffe Mono-, Di- oder Triglyceride der nachstehenden Formeln

$$\begin{array}{l} CH_2-O-CO-R_1 \\ | \\ CH-OH \\ | \\ CH_2-OH \end{array} , \qquad \begin{array}{l} CH_2-O-CO-R_1 \\ | \\ CH-O-CO-R_2 \\ | \\ CH_2-OH \end{array} , \qquad \begin{array}{l} CH_2-O-CO-R_1 \\ | \\ CH-O-CO-R_2 \\ | \\ CH_2-O-CO-R_3 \end{array}$$

(eingeschlossen Strukturisomere) oder Mischungen davon, die zu Nitrilen der Formeln $R_1$-CN, $R_2$-CN und $R_3$-CN führen.

In diesen Formeln können $R_1$, und $R_2$ oder $R_1$, $R_2$ und $R_3$ gleich sein oder verschieden sein im Falle von Di- und Triglyceriden. Die Reste $R_1$, $R_2$ und $R_3$ sind ausgewählt aus den folgenden Gruppen:

a) Alkylradikale, die verzweigt sein können, aber vorzugsweise geradkettig sind, mit 3 bis 23, vorzugsweise 7 bis 23 C-Atomen;

b) olefinisch ungesättigte aliphatische Kohlenwasserstoffreste, die verzweigt sein können, aber vorzugsweise geradkettig sind, mit 3 bis 23, vorzugsweise 11 bis 21 und insbesondere 15 bis 21 C-Atomen, und mit 1 bis 6, vorzugsweise 1 bis 3 Doppelbindungen, die konjugiert oder isoliert sein können; und

c) monohydroxy-substituierte Reste des Typs a) und b), vorzugsweise die ungesättigten Kohlenwasserstoffreste, die 1 bis 3 Doppelbindungen besitzen, insbesondere den Rest der Ricinolsäure.

Die Acylradikale $R_1$-CO-, $R_2$-CO- und $R_3$-CO-solcher Glyceride, die als Ausgangsmaterialien für das Verfahren der vorliegenden Erfindung geeignet sind, leiten sich ab von den folgenden Gruppen aliphatischer Carbonsäuren (Fettsäuren):

a) Alkansäuren und deren alkylverzweigte, vorzugsweise methylverzweigte Derivate, mit 4 bis 24 Kohlenstoffatomen, wie zum Beispiel Buttersäure, Valeriansäure, Capronsäure, Heptansäure, Caprylsäure, Pelargonsäure, Caprinsäure, Undecansäure, Laurinsäure, Tridecansäure, Myristinsäure, Pentadecansäure, Palmitinsäure, Margarinesäure, Stearinsäure, Nonadecansäure, Arachinsäure, Behensäure, Lignocerinsäure, 2-Methylbutansäure, Isobuttersäure, Isovaleriansäure, Pivalinsäure, Isocapronsäure, 2-Ethylcapronsäure, die stellungsisomeren Methylcaprinsäuren, Methyllaurinsäuren und Methylstearinsäuren, 12-Hexylstearinsäure, Isostearinsäure oder 3,3-Dimethylstearinsäure;

b) Alkensäuren, Alkadiensäuren, Alkatriensäuren, Alkatetraensäuren, Alkapentaensäuren und Alkahexaensäuren und deren alkylverzweigte, vorzugsweise methylverzweigte Derivate, mit 4 bis 24 C-Atomen, wie zum Beispiel Crotonsäure, Isocrotonsäure, Caproleinsäure, Linderinsäure, Lauroleinsäure, Myristoleinsäure; Palmitoleinsäure, Petroselinsäure, Ölsäure, Elaidinsäure, Erucasäure, Brassidinsäure, 2,4-Decadiensäure, Linoleinsäure, 11,14-Eicosadiensäure, Hiragonsäure, Eleostearinsäure, Linolensäure, Pseudoeleostearinsäure, Arachidonsäure, 4,8,12,15,18,21-Tetracosahexaensäure oder trans-2-Methyl-2-butensäure;

c) Monohydroxyalkansäuren mit 4 bis 24 C-Atomen, vorzugsweise 12 bis 24 C-Atomen, vorzugsweise unverzweigt, wie zum Beispiel Hydroxybuttersäure, Hydroxyvaleriansäure, Hydroxycapronsäure, 2-Hydroxydodecansäure, 2-Hydroxytetradecansäure, 15-Hydroxypentadecansäure, 16-Hydroxyhexadecansäure oder 18-Hydroxyoctadecansäure; und

d) Monohydroxyalkensäuren mit 4 bis 24, vorzugsweise mit 12 bis 22 und insbesondere mit 16 bis 22 C-Atomen (vorzugsweise unverzweigt) und mit 1 bis 6, vorzugsweise mit 1 bis 3 und insbesondere mit einer ethylenischen Doppelbindung, wie zum Beispiel Ricinoleinsäure oder Ricinelaidinsäure.

Bevorzugte Ausgangsstoffe für das erfindungsgemäße Verfahren sind vor allem die natürlichen (pflanzlichen) oder tierischen Fette und Öle, die Gemische aus überwiegend Triglyceriden und kleinen Anteilen aus Diglyceriden und/oder Monoglyceriden darstellen, wobei auch diese Glyceride meist wiederum Gemische darstellen und verschiedenartige Fettsäurereste im obengenannten Bereich, insbesondere solche mit 8 und mehr C-Atomen, enthalten. Beispielsweise seien genannt pflanzliche Fette und Öle, wie Olivenöl, Kokosfett, Palmkernfett, Babassuöl, Palmöl, Erdnußöl, Rüböl, Ricinusöl, Sesamöl, Cottonöl, Sonnenblumenöl, Sojaöl, Hanföl, Mohnöl, Avocadoöl, Baumwollsaatöl, Weizenkeimöl, Maiskaimöl, Kürbiskernöl, Traubenkernöl, Kakaobutter oder auch Pflanzentalge, ferner tierische Fette und Öle, wie Rindertalg, Schweinefett, Knochenfett, Hammeltalg, Japantalg, Walöl und andere Fischöle sowie Lebertran. Ebenso eingesetzt werden können einheitliche Tri-, Di- und Monoglyceride oder Mischungen davon, sei es, daß diese aus natürlichen Fetten isoliert oder auf synthetischem Wege gewonnen wurden. Hier seien beispielsweise genannt: Tributyrin, Tricapronin, Tricaprylin, Tricaprinin, Trilaurin, Trimyristin, Tripalmitin, Tristearin, Triolein, Trielaidin, Trilinolein, Trilinolenin, Monopalmitin, Monostearin, Monoolein, Monocaprinin, Monolaurin, Monomyristin oder gemischte Glyceride, beispielsweise Palmitodistearin, Distearoolein, Dipalmitoolein und Myristopalmitostearin.

Die erfindungsgemäß einzusetzenden Katalysatoren sind, ebenso wie beim bekannten Verfahren, ausgewählte spezielle Katalysatoren, wie sie in den eingangs genannten Druckschriften, US-Patentschrift 4 234 509 und DE 32 44 752 A1, beschrieben sind. Es handelt sich um Katalysatoren ausgewählt aus der Gruppe bestehend aus a) Metallsalzen von Carbonsäuren oder Sulfonsäuren, wobei in dem Metallsalz das Metallkation Antimon, Blei, Cadmium, Chrom, Eisen, Kobalt, Mangan, Nickel, Titan, Zink, Zinn oder Zirkon ist, vorzugsweise Blei, Cadmium, Eisen, Kobalt oder Zink, und b) Diorganozinn(IV)-bis-sulfonaten der Formel

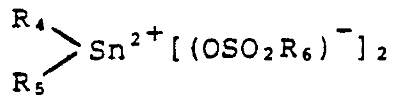 ,

worin $R_4$, $R_5$ und $R_6$, gleich oder verschieden, einen Alkylrest, Arylrest, alkylsubstituierten Arylrest, Aralkylrest oder einen Cycloalkylrest bedeuten.

Das Carbonsäure- oder Sulfonsäure-Anion der genannten Metallsalze stammt aus einer der folgenden Gruppen: (1) gesättigte aliphatische Mono- oder Polycarbonsäuren, vorzugsweise die Monocarbonsäuren, mit 4 bis 24, vorzugsweise 8 bis 24 C-Atomen, die verzweigt sein können, aber vorzugsweise geradkettig sind; (2) olefinisch ungesättigte aliphatische Mono- oder Polycarbonsäuren, vorzugsweise die Monocarbonsäuren, mit 1 bis 6, vorzugsweise 1 bis 3, isolierten oder konjugierten Doppelbindungen und mit 4 bis 24, vorzugsweise 8 bis 24 C-Atomen, die verzweigt sein können, aber vorzugsweise geradkettig sind; (3) mono-, di- oder trialkylierte, vorzugsweise mono- oder dialkylierte, Benzol- oder Naphthalinmono- oder -polycarbonsäuren, vorzugsweise die Monocarbonsäuren, mit Alkylresten mit 1 bis 24, vorzugsweise 1 bis 12 C-Atomen, wobei diese Alkylreste verzweigt sein können, aber vorzugsweise geradkettig sind; (4) Alkanmono- oder Alkandisulfonsäuren, vorzugsweise die Monosulfonsäuren, mit 4 bis 24, vorzugsweise 8 bis 24 C-Atomen, die verzweigt sein können, aber vorzugsweise geradkettig sind; (5) fluor-substituierte Alkanmonosulfonsäuren, vorzugsweise perfluor-substituierte, mit 4 bis 24, vorzugsweise 8 bis 24 C-Atomen, die verzweigt sein können, aber vorzugsweise geradkettig sind; (6) monohydroxy-substituierte Alkanmonosulfonsäuren mit 4 bis 24, vorzugsweise 8 bis 24 C-Atomen, die verzweigt sein können, aber vorzugsweise geradkettig sind; (7) mono-, di- oder trialkylierte, vorzugsweise mono- oder dialkylierte, Benzol- oder Naphthalinmono- oder -polysulfonsäuren, vorzugsweise die Monosulfonsäuren, mit Alkylresten mit 1 bis 24, vorzugsweise 1 bis 12 C-Atomen, wobei diese Alkylreste verzweigt sein können, aber vorzugsweise geradkettig sind; und (8) (monocarboxy)alkyl-oder (dicarboxy)alkyl-substituierte Benzol- oder Naphthalinmono-oder -polysulfonsäuren, vorzugsweise die (monocarboxy)alkyl-substituierten Monosulfonsäuren, mit Alkylresten mit 1 bis 24, vorzugsweise 1 bis 12 C-Atomen.

Bezüglich der Diorganozinnsulfonate der obengenannten Formel sind solche bevorzugt, in denen $R_4$, $R_5$ und $R_6$ die nachstehend angebenen Bedeutungen haben: $R_4$ und $R_5$, die vorzugsweise gleich sind, bedeuten geradkettige oder verzweigte Alkylreste mit 1 bis 22, vorzugsweise 4 bis 18 C-Atomen; Arylreste, vorzugsweise Phenyl- oder Naphthylreste, die mit 1 bis 3 geradkettigen oder verzweigten Alkylresten mit jeweils 1 bis 22, vorzugsweise 1 bis 12 C-Atomen, substituiert sein können, wobei die monosubstituierten Arylreste bevorzugt sind; Aralkylreste, vorzugsweise den Benzylrest; oder Cycloalkylreste, vorzugsweise den Cyclohexylrest. $R_6$, das von $R_4$ und $R_5$ verschieden oder mit einem oder beiden dieser Reste gleich sein kann, bedeutet einen Alkylrest mit 4 bis 24, vorzugsweise 8 bis 24 C-Atomen, der geradkettig oder verzweigt sein kann; einen Arylrest, vorzugsweise den Phenyl- oder den Naphthylrest, der mit 1 bis 3, vorzugsweise mit einem geradkettigen oder verzweigten Alkylrest mit 1 bis 22, vorzugsweise 1 bis 12 C-Atomen, substituiert sein kann; oder einen Aralkylrest, vorzugsweise den Benzylrest.

Im Falle von Metallsalzen von Carbonsäuren oder Sulfonsäuren als Katalysatoren sind solche besonders bevorzugt, bei denen das Metallkation Blei, Cadmium, Eisen, Kobalt oder Zink ist und das Anion ausgewählt ist aus den oben angeführten Vertretern (4), (6) und (7), wobei die Alkanmonosulfonsäuren und die Alkylarylsulfonsäuren bevorzugt sind, so zum Beispiel die n-Octansulfonsäure, n-Dodecylsulfonsäure, n-Octadecylsulfonsäure, $C_{15}$-$C_{18}$-Alkansulfonsäure, Talgfettsäure, Methylbenzol(Toluol)sulfonsäure, n-Hexylbenzolsulfonsäure, n-Dodecylbenzolsulfonsäure, n-Butylnaphthalinsulfonsäure und n-Dodecylnaphthalinsulfonsäure. Im Falle von Diorganozinnsulfonaten als Katalysatoren sind solche besonders bevorzugt, die sich aus der obigen Formel ergeben, wenn $R_4$ und $R_5$, die vorzugsweise gleich sind, einen Alkylrest mit 1 bis 22, vorzugsweise 4 bis 18 C-Atomen, einen Phenylrest oder einen Naphthylrest bedeuten, und $R_6$ ein Alkylrest mit 4 bis 24, vorzugsweise 8 bis 24 C-Atomen, oder ein Arylrest ist, der aus einem unsubstituierten Phenylrest oder einem mit einem Alkylrest mit 1 bis 22, vorzugsweise 1 bis 12 C-Atomen, substituierten Phenylrest besteht.

Die genannten Katalysatoren werden in einer Menge von 0,5 bis 10 Gew.-%, vorzugsweise 1 bis 5 Gew.-%, bezogen auf das Glycerid, eingesetzt. Sie werden in der Regel als solche dem Glycerid zugegeben. Es kann auch das entsprechende Metalloxid und die entsprechende Carbonsäure oder Sulfonsäure einzeln zugegeben werden, wobei sich dann dieser Katalysator während der Reaktion in situ bildet. Ebenso können im Falle des Organozinnkatalysators die Ausgangs-Organozinnverbindungen (Oxide, Hydroxide, fettsaure Salze) und die freien Sulfonsäuren zugegeben werden, wobei sich dann die Diorganozinn(IV)-bis-sulfonat-Verbindungen ebenfalls in situ bilden.

Was die an sich bekannten Maßnahmen des erfindungsgemäßen Verfahrens betrifft, so beträgt auch hier die Menge an Ammoniak, die durch das im Reaktionsgefäß erhitzte Glycerid hindurchgeleitet wird, mindestens 200 Liter pro Kilogramm Glycerid pro Stunde, vorzugsweise mindestens 400 Liter pro Kilogramm Glycerid pro Stunde. Nach oben besteht keine kritische Grenze bezüglich des durchzuleitenden Ammoniakstromes, die mengenmäßige Obergrenze wird allenfalls durch wirtschaftliche Überlegungen bestimmt und liegt aus diesen Gründen bei etwa 1000 Liter, vorzugsweise bei etwa 800 Liter Ammoniak pro Kilogramm Glycerid pro Stunde. Dem Ammoniak, dessen Menge also im allgemeinen bei 200 bis 1000 Liter, vorzugsweise 400 bis 800 Liter pro Kilogramm Glycerid pro Stunde liegt, kann mit Vorteil bis zu 30 Vol.-%, vorzugsweise bis zu 15 Vol.-%, bezogen auf die durchgesetzte Menge an Ammoniak, an Inertgas, beispielsweise Stickstoff, hinzugefügt werden. Mit dem Durchleiten des genannten Ammoniakstromes durch das auf 220 bis 300 °C erhitzte (und damit in flüssiger Phase vorliegende) Glycerid ist ein guter Kontakt zwischen Glycerid und Ammoniak gegeben und auch eine rasche Austragung des gebildeten Glycerins und ebenso des gebildeten Roh-Fettsäurenitrils und Reaktionswassers aus dem Reaktionsgefäß.
Das Glycerid ist, wie bereits erwähnt, auf einer Temperatur von 220 bis 300 °C, vorzugsweise 230 bis 270 °C erhitzt. Diese Reaktionstemperatur wird während der gesamten Glyceridumsetzung gehalten, das heißt so lange, bis das vorgelegte Glycerid praktisch kein Glycerin mehr abgibt (Ende der Hauptreaktion). Es ist bevorzugt, die Temperatur vom Beginn bis zum Ende der Umsetzung ansteigen zu lassen, entweder kontinuierlich oder stufenweise, insbesondere in Form eines Temperaturprogramms. Nach einer bevorzugten Ausführungsform wird die Umsetzung (Hauptreaktion) zunächst im Temperaturbereich von etwa 220 bis 240 °C so lange durchgeführt, bis etwa 30 bis 70 Gew.-% der theoretisch zu erwartenden Glycerinmenge aus dem Reaktionsgefäß ausgetragen sind. Dann wird die Temperatur im Verlauf von etwa 1/2 bis 5 Stunden stufenweise oder kontinuierlich auf etwa 250 bis 270 °C erhöht, worauf die Reaktion bei der erhöhten Temperatur zu Ende geführt wird. Das Ende der Reaktion ist daran erkennbar, daß kein oder praktisch kein Glycerin mehr in die Vorlage übergeht.
Nach Beendigung der Hauptreaktion werden, ebenso wie beim bekannten Verfahren die Bedingungen der Nachreaktion eingestellt. Das im Reaktionsgefäß befindliche gesamte Roh-Fettsäurenitril (das ist das im Laufe der Hauptreaktion erfindungsgemäß zurückgeführte und das im Reaktionsgefäß verbliebene, Fettsäure und Fettsäureamid enthaltende Fettsäurenitril) wird auf eine Temperatur von 240 bis 320 °C, vorzugsweise 260 bis 300 °C, gebracht, und es wird ein Ammoniakstrom von 5 bis 150 Liter, vorzugsweise 15 bis 100 Liter pro Kilogramm Roh-Fettsäurenitril pro Stunde eingestellt und durch den Inhalt des Reaktionsgefäßes (der in flüssiger Phase vorliegt) hindurchgeleitet. Mit diesen Bedingungen an Reaktionstemperatur und Ammoniakmenge werden in Gegenwart der für die Hauptreaktion eingesetzten Katalysatoren die im Roh-Fettsäurenitril enthaltenen Fettsäuren und Fettsäureamide in Fettsäurenitrile umgesetzt, wobei das entstehende Reaktionswasser aus dem Reaktionsgefäß ausgetragen wird. Sofern mit dem Ammoniakstrom auch Fettsäurenitrile, Fettsäuren und/oder Fettsäureamide ausgetragen werden sollten, werden diese Komponenten wieder in das Reaktionsgefäß zurückgeführt. Sind für die Hauptreaktion bereits genügend Katalysatoren eingesetzt worden (eine Katalysatormenge, die an der oberen Grenze der oben angeführten Mengenbereiche liegt) so erübrigt sich die neuerliche Zugabe von Katalysator. Andernfalls ist eine entsprechende Menge an Katalysator hinzuzufügen. Die Menge an Katalysator bei der Nachreaktion liegt im Bereich von 0,5 bis 10 Gew.-%, vorzugsweise 1 bis 5 Gew.-%, bezogen auf das Gewicht des Roh-Fettsäurenitrils. Bei diesen Reaktionsbedingungen geht die vollständige Umwandlung von Fettsäureamiden und Fettsäuren in Fettsäurenitrile vor sich, die an der Bildung von Reaktionswasser, das mit dem überschüssigen Ammoniak ausgetragen wird, verfolgt werden kann. Ebenso wie bei der Hauptreaktion kann der überschüssige Ammoniakstrom, der von Reaktionskomponenten und Reaktionswasser zweckmäßigerweise vollständig befreit worden ist, gegebenenfalls nach Zugabe von frischem Ammoniak, wieder in die Reaktion zurückgeführt werden. Nach Beendigung der Nachreaktion kann das im Reaktionsgefäß vorliegende und angestrebte Fettsäurenitril abgezogen werden. Wie bereits oben erwähnt, kann der in diesem an sich reinen Fettsäurenitril noch vorhandene Katalysator beispielsweise durch Destillation abgetrennt werden.

Die Erfindung wird nun an Beispielen noch näher erläutert.

Beispiel 1

In diesem erfindungsgemäßen Beispiel wurde Roh-Fettsäurenitril während der Glyceridumsetzung kontinuierlich in die Reaktion zurückgeführt. 500 g Tierkörperfett (Verseifungszahl 185, Säurezahl 13,9) und 10 g Zink-Dodecylbenzolsulfonat als Katalysator (das sind 2 Gew.-% Katalysator, bezogen auf das Fett oder Glycerid) wurden in einem Reaktionsgefäß vorgelegt. Das 700 ml fassende Reaktionsgefäß war ausgestattet mit einer Heizeinrichtung zum Erhitzen des Inhaltes auf Reaktionstemperatur, einem Rührer zum Rühren des Inhaltes, mit einem Innenthermometer zur Feststellung der vorliegenden Temperatur und mit einer

Gaseinleitung für den Ammoniakstrom. Das Reaktionsgefäß war mit einer Kühlkolonne (einem Kondensator) verbunden, die höher als das Reaktionsgefäß und seitlich davon angeordnet war. Die Leitung vom Kopf des Reaktionsgefäßes in den unteren Teil der Kühlkolonne war mit einer elektrischen Heizung ausgestattet. Der Boden der Kühlkolonne war mit einem zylinderförmigen, 100 ml fassenden Gefäß zur Aufnahme und Abscheidung der kondensierten Phasenkomponenten verbunden. Von dem Gefäß, das ist die Vorlage, führte eine Leitung zum Reaktionsgefäß zurück. Diese Leitung ging vom Kopf der Vorlage zum Kopf des Reaktionsgefäßes und war zum Reaktionsgefäß hin geneigt, so daß ein Fluß von der Vorlage zum Reaktionsgefäß erfolgen konnte.

Während des Aufheizens wurde das Reaktionsgefäß mit Stickstoff gespült. Bei 150 °C wurde der Stickstoff durch Ammoniakgas ersetzt, das in einer Menge von 600 Liter Ammoniak pro Kilogramm Tierkörperfett (Talg) pro Stunde im Kreis geführt wurde. Das Reaktionsgefäß und damit der Inhalt des Reaktionsgefäßes wurde auf 230 °C erhitzt (Beginn der Glyceridumsetzung oder Hauptreaktion) und bei dieser Temperatur 3 Stunden lang gehalten. Während der Reaktion wurde laufend frisches gasförmiges Ammoniak zugegeben, um die angegebenen 600 Liter Ammoniak aufrecht zu halten. Nach den 3 Stunden Reaktionszeit wurde die Temperatur innerhalb von etwa 1,5 Stunden von 230 auf 260 °C erhöht, und es wurde die Temperatur von 260 °C 30 Minuten lang gehalten. Nach dieser Zeit, das sind 5 Stunden reaktionszeit, war praktisch kein Glycerin mehr im ausgetragenen Produktgemisch, was das Ende der Umsetzung vom eingesetzen Talg mit Ammoniak anzeigte (Ende der Glyceridumsetzung oder der Hauptreaktion). Während der Glyceridumsetzung, die bei der Temperatur von 230 °C begann, wurde das gebildete Produktgemisch, bestehend im wesentlichen aus Glycerin, Roh-Fettsäurenitril und Wasser, mit dem überschüssigen Ammoniakstrom über die auf zunächst 230 °C und dann bis auf 260 °C erhitzte Verbindungsleitung vom Reaktionsgefäß zur Kühlkolonne aus dem Reaktionsgefäß in die Kühlkolonne ausgetragen (mit dem erfindungsgemäß gezielten Erhitzen der in Rede stehenden Verbindungsleitung wird erreicht, daß Kondensation von Glycerin in dieser Leitung und damit Rückfluß von Glycerin in das Reaktionsgefäß ausgeschlossen ist). Die beiden Komponenten Glycerin und Roh-Fettsäurenitril (dieses enthielt etwa 2 Gew.-% Fettsäure und 3 Gew.-% Fettsäureamid) kondensierten in der Kühlkolonne praktisch vollständig und sammelten sich in der Vorlage unter Abscheidung einer unteren Glycerin-Phase und einer oberen Roh-Fettsäurenitril-Phase. Das Ammoniak und das im wesentlichen gesamte Wasser verließen die Kühlkolonne gasförmig (das Ammoniak wurde nach Abtrennung des Wassers wieder in das Reaktionsgefäß zurückgeführt). Die Vorlage und damit ihr Inhalt wurde bei einer Temperatur von 150 °C gehalten, wodurch noch gegebenenfalls vorhandenes Restwasser und gelöstes Ammoniak entfernt wurde. Die Glycerin-Phase wurde mit Hilfe eines Magnetrührers in Bewegung gehalten um die genannte Entgasung von Ammoniak zu beschleunigen und zu vervollständigen.

Die 100 ml fassende Vorlage war nach etwa 30 min Reaktionszeit bis zur Ansatzstelle der Verbindungsleitung zum Reaktionsgefäß (das heißt bis zum Überlauf) mit etwa 90 ml angefüllt. In dieser Zeit waren etwa 15 Vol-% Glycerin plus Roh-Fettsäurenitril, bezogen auf die zu erwartende Gesamtmenge an Glycerin und Roh-Fettsäurenitril, ausgetragen (die 90 ml bestanden aus etwa 8 ml Glycerin und 82 ml Roh-Fettsäurenitril). Nach Auffüllung der Vorlage setzte zwangsläufiger Rückfluß von Roh-Fettsäurenitril über die Verbindungsleitung in das Reaktionsgefäß ein. Ab nun floß also kontinuierlich im wesentlichen die gleiche Menge an Roh-Fettsäurenitril in die Reaktion zurück, die während der Umsetzung des Fettes mit Ammoniak gebildet und mit dem Ammoniakstrom gemeinsam mit dem Glycerin und dem Reaktionswasser ausgetragen wurde (die Verbindungsleitung von der Vorlage zum Reaktionsgefäß war auf jene Temperatur erhitzt, die der Reaktionstemperatur entsprach, um Abkühlung des Rückflusses durch die Verbindungsleitung und damit eventuell Abkühlung im Reaktionsgefäß zu vermeiden). Nach dem Ende der Glyceridumsetzung, das heißt nach den genannten 5 Stunden Reaktionszeit, wurde das angestrebte Glycerin von der Vorlage abgezogen. Die in der Vorlage befindliche Menge an Roh-Fettsäurenitril wurde in das Reaktionsgefäß gegeben (diese Menge an Roh-Fettsäurenitril ist im Vergleich zur gesamten Menge an Roh-Fettsäurenitril sehr gering). Die Temperatur des Reaktionsgefäßes, in dem die gesamte Menge an Roh-Fettsäurenitril vorlag, wurde auf 300 °C erhöht und der Ammoniakstrom auf 100 Liter Ammoniak pro Kilogramm Roh-Fettsäurenitril pro Stunde reduziert. Diese Bedingungen (das sind die Bedingungen der Nachreaktion) wurden 1,5 Stunden lang gehalten. Das in der Nachreaktion gebildete Reaktionswasser wurde mit dem überschüssigen Ammoniakstrom über die nun nicht mehr beheizte Verbindungsleitung und die Kühlkolonne ausgetragen. Trotz des geringen Ammoniakstromes eventuell mitgeführtes Fettsäurenitril kondensierte bereits in der nicht mehr beheizten Verbindungsleitung und floß in das Reaktionsgefäß zurück (die Vorlage wurde zu Beginn der Nachreaktion vom Reaktionsgefäß und von der Kühlkolonne abgetrennt, da sie nicht mehr benötigt wurde). Nach der genannten Reaktionszeit von 1,5 Stunden waren die umzuwandelnden Komponenten, Fettsäure und Fettsäureamid, in Fettsäurenitril umgewandelt. Das an sich reine, nur noch Katalysator enthaltende, Fettsäurenitril wurde vom Reaktionsgefäß abgezogen und in einer Destillation vom

Katalysator befreit.
Es wurden die folgenden Ausbeuten und Reinheiten erreicht:
Glycerin-Ausbeute: 38 ml (45 g), das sind 90 Gew.-% der Theorie; das Glycerin war praktisch frei von Wasser, Fettsäure, Fettsäureamid und Fettsäurenitril und stellte somit ohne irgendeine weitere Reinigungsoperation bereits ein Reinglycerin dar. Fettsäurenitril-Ausbeute: 533 ml (426 g), das sind 95 Gew.-% der Theorie; das Fettsäurenitril war frei von Glycerin und Wasser und enthielt nur noch 0,25 Gew.-% Fettsäureamid und 0,2 Gew.-% Fettsäure.

Beispiel 2

In diesem erfindungsgemäßen Beispiel wurde Roh-Fettsäurenitril während der Glyceridumsetzung stufenweise in die Reaktion zurückgeführt.

Das Beispiel wurde mit den gleichen Reaktionskomponenten und mit den gleichen Reaktionsbedingungen durchgeführt wie das Beispiel 1, mit dem Unterschied, daß das während der Glyceridumsetzung gebildete und entfernte Roh-Fettsäurenitril nicht kontinuierlich, sondern portionsweise in die Reaktion zurückgebracht wurde. Zu diesem Zweck war die in Beispiel 1 verwendete Apparatur in der Weise geändert, daß eine Vorlage von 200 ml Inhalt genommen wurde, und daß die Verbindungsleitung von der Vorlage zum Reaktionsgefäß nicht am Kopf der Vorlage, sondern in ihrem unteren Teil ansetzte und zudem eine Absperreinrichtung hatte.
Nachdem die Vorlage bis zum Überlauf gefüllt war (vom Beginn der Glyceridumsetzung bis zu diesem Zeitpunkt waren 15 Minuten vergangen und es waren etwa 5 ml Glycerin und etwa 40 ml Roh-Fettsäurenitril, das sind etwa 8 Vol.-% von der insgesamt zu erwartenden Menge an Glycerin plus Roh-Fettsäurenitril, ausgetragen und in der Vorlage abgeschieden), und nachdem etwa 30 Minuten lang zwangsläufiger Rückfluß von Roh-Fettsäurenitril gehalten wurde, wurde der Rückfluß mit Hilfe der genannten Absperreinrichtung gestoppt, so daß sich das Roh-Fettsäurenitril in der Vorlage ansammelte. Nach 15 Minuten hatten sich etwa 40 ml Roh-Fettsäurenitril angesammelt. Diese Menge an Roh-Fettsäurenitril wurde nun durch Öffnen der Absperreinrichtung innerhalb von etwa 5 Minuten in das Reaktionsgefäß zurückfließen gelassen (zudosiert), worauf die Absperreinrichtung zur Ansammlung der gleichen Menge an Roh-Fettsäurenitril wieder geschlossen wurde. Die beschriebene Vorgangsweise der Ansammlung und stufenweisen (portionsweisen) Rückführung von Roh-Fettsäurenitril wurde bis zum Ende der Glyceridumsetzung, das nach 5 Stunden erreicht war, noch 9mal wiederholt. Nach dem Ende der Glyceridumsetzung wurde wie in Beispiel 1 weiter vorgegangen.

Es wurden die folgenden Ausbeuten und Reinheiten erreicht:
Glycerinausbeute: 38 ml (45 g), das sind 90 Gew.-% der Theorie; das Glycerin war praktisch frei von Wasser, Fettsäure, Fettsäureamid und Fettsäurenitril und stellte somit ohne irgendeine weitere Reinigungsoperation bereits ein Reinglycerin dar. Fettsäurenitril-Ausbeute: 533 ml (426 g), das sind 95 Gew.-% der Theorie; das Fettsäurenitril war frei von Glycerin und Wasser und enthielt nur noch 0,25 Gew.-% Fettsäureamid und 0,2 Gew.-% Fettsäure.

Das in den Beispielen 1 und 2 eingesetzte Tierkörperfett war eine Mischung aus im wesentlichen Rindertalg, Hammeltalg, Schweinefett und Knochenfett.

Beispiele 3 bis 7

Diese Beispiele wurden analog Beispiel 1 durchgeführt, jedoch mit anderen Glyceriden, Katalysatoren, Ammoniak ($NH_3$)-Mengen, Reaktionstemperaturen und/oder Reaktionszeiten. Im folgenden sind diese Variationen und die Ergebnisse der Beispiele im einzelnen angegeben (alle Prozentangaben sind Gewichtsprozente; von den beiden Zahlen, die nach dem eingesetzten Glycerid in Klammern angeführt sind, ist die erste die Verseifungszahl und die zweite die Säurezahl; die Prozentangabe nach der Katalysatorverbindung ist die eingesetzte Menge an Katalysator).

Beispiel 3

Technischer Rindertalg (186; 12,6); Blei-n-Dodecylsulfonat, 3 %; 400 l $NH_3$ während der Glyceridumsetzung und 100 l $NH_3$ während der Nachreaktion; Reaktionstemperatur bei der Glyceridumsetzung 250 °C 3 Stunden und bei der Nachreaktion 280 °C 1,5 Stunden. Ergebnis: Glycerinausbeute 86 %, Fettsäurenitril-Ausbeute 93 % mit 0,13 % Fettsäureamid und 0,12 % Fettsäure.

Beispiel 4

Speisetalg (190; 2,4); Cadmium-n-Octadecylsulfonat, 2 %; 800 l $NH_3$ während der Glyceridumsetzung und 100 l $NH_3$während der Nachreaktion; Reaktionstemperatur bei der Glyceridumsetzung 230 °C 3 Stunden, anschließend Erhöhung auf 260 °C innerhalb von 1,5 Stunden (5 °C pro 15 Minuten), Reaktionstemperatur bei der Nachreaktion 300 °C 1,5 Stunden. Ergebnis: Glycerinausbeute 91 %, Fettsäurenitril-Ausbeute 95 % mit weniger als 0,1 % Fettsäureamid und weniger als 0,1 % Fettsäure.

Beispiel 5

Schweinefett (124; 11,7); Kobalt-n-Octansulfonat, 4 %; 400 l $NH_3$ während der Glyceridumsetzung und 15 l $NH_3$ während der Nachreaktion; Reaktionstemperatur bei der Gylceridumsetzung 230 °C 3 Stunden, anschließend Erhöhung auf 250 °C innerhalb von 1 Stunde (5 °C pro 15 Minuten), Reaktionstemperatur bei der Nachreaktion 275 °C 2 Stunden. Ergebnis: Glycerinausbeute 87 %, Fettsäurenitril-Ausbeute 92 % mit 0,22 % Fettsäureamid und 0,20 % Fettsäure.

Beispiel 6

Sonnenblumenöl (189; 0,8); Eisen(II)-n-Butylnaphthalinsulfonat, 5 %; 400 l $NH_3$ während der Glyceridumsetzung und 50 l $NH_3$ während der Nachreaktion; Reaktionstemperatur bei der Glyceridumsetzung 240 °C 4 Stunden und bei der Nachreaktion 300 °C 1,5 Stunden. Ergebnis: Glycerinausbeute 87 %, Fettsäurenitril-Ausbeute 91 % mit 0,20 % Fettsäureamid und 0,15 % Fettsäure.

Beispiel 7

Sojaöl (203; 0,5); Di-n-octyl-Zinn(IV)-Bis-(toluolsulfonat), 2 %; 800 l $NH_3$ während der Glyceridumsetzung und 50 l $NH_3$ während der Nachreaktion; Reaktionstemperaturen wie in Beispiel 5. Ergebnis: Glycerinausbeute 89 %, Fettsäurenitril-Ausbeute 94 % mit 0,18 % Fettsäureamid und 0,10 % Fettsäure.

Beispiele 8 bis 13

Diese Beispiele wurden analog Beispiel 2 durchgeführt, jedoch mit anderen Glyceriden, Katalysatoren, Ammoniak ($NH_3$)-Mengen, Reaktionstemperaturen und/oder Reaktionszeiten. Im folgenden sind diese Variationen und die Ergebnisse der Beispiele im einzelnen angegeben (alle Prozentangaben sind Gewichtsprozente; von den beiden Zahlen, die nach dem eingesetzten Glycerid in Klammern angeführt sind, ist die erste die Verseifungszahl und die zweite die Säurezahl; die Prozentangabe nach der Katalysatorverbindung ist die eingesetzte Menge an Katalysator).

Beispiel 8

Palmöl (202; 0,7); Zink-$C_{15}/C_{18}$-Alkansulfonat, 3 %; 400 l $NH_3$ während der Glyceridumsetzung und 15 l $NH_3$ während der Nachreaktion; Reaktionstemperaturen wie in Beispiel 5. Ergebnis: Glycerinausbeute 92 %, Fettsäurenitril-Ausbeute 95 % mit 0,28 % Fettsäureamid und 0,23 % Fettsäure.

Beispiel 9

Cocosfett (240; 1,8); Bleisalz der Talgfettsäure, 5 %; 600 l $NH_3$ während der Glyceridumsetzung und 100 l während der Nachreaktion; Reaktionstemperaturen wie in Beispiel 6. Ergebnis: Glycerinausbeute 84 %, Fettsäurenitril-Ausbeute 94 % mit 0,24 % Fettsäureamid und 0,15 % Fettsäure.

Beispiel 10

Ricinusöl (173; 1,8); Cadmium-Methylbenzol(toluol)sulfonat, 3 %; 800 l $NH_3$ während der Glyceridumsetzung und 50 l $NH_3$ während der Nachreaktion; Reaktionstemperaturen wie in Beispiel 3. Ergebnis: Glycerinausbeute 74 %, Fettsäurenitril-Ausbeute 65 % mit 0,20 % Fettsäureamid und 0,18 % Fettsäure.

Beispiel 11

Rüböl (167; 1,8); Kobalt-n-Dodecylnaphthalinsulfonat, 4 %; 600 l $NH_3$ während der Glyceridumsetzung und 50 l $NH_3$ während der Nachreaktion; Reaktionstemperaturen wie in Beispiel 6. Ergebnis: Glycerin-Ausbeute 89 %, Fettsäurenitril-Ausbeute 91 % mit 0,16 % Fettsäureamid und 0,12 % Fettsäure.

Beispiel 12

Walöl (189; 6,3); Eisen-Hexylbenzolsulfonat, 4 %; 400 l $NH_3$ während der Glyceridumsetzung und 50 l $NH_3$ während der Nachreaktion; Reaktionstemperaturen wie in Beispiel 3. Ergebnis: Glycerinausbeute 83 %, Fettsäurenitril-Ausbeute 86 % mit 0,27 % Fettsäureamid und 0,23 % Fettsäure.

Beispiel 13

Technischer Rindertalg (186; 12,6); Di-n-butyl-Zinn(IV)-Bis-(n-dodecylbenzolsulfonat), 2 %; 800 l $NH_3$ während der Glyceridumsetzung und 100 l $NH_3$ während der Nachreaktion. Reaktionstemperatur bei der Glyceridumsetzung 230 °C 3 Stunden, anschließend Erhöhung auf 260 °C innerhalb von 1,5 Stunden (5 °C pro 15 Minuten) und 0,5 Stunden bei 260 °C, Reaktionstemperatur bei der Nachreaktion 300 °C 1,5 Stunden (das gleiche Temperaturprogramm wurde in den Beispielen 1 und 2 eingehalten). Ergebnis: Glycerin-Ausbeute 93 %, Fettsäurenitril-Ausbeute 96 % mit 0,14 % Fettsäureamid und 0,10 % Fettsäure.

Beispiele 14 bis 17

Diese Beispiele wurden analog Beispiel 1 durchgeführt, jedoch mit Mono- oder Triglyceriden als Glycerid, mit Diorganozinn(IV)-Bis-sulfonaten als Katalysatoren und mit anderen Ammoniak-Mengen, Reaktionstemperaturen und/oder Reaktionszeiten. Im folgenden sind diese Variationen und die Ergebnisse der Beispiele im einzelnen angegeben (die bei den Beispielen 3 bis 7 an dieser Stelle angegebenen Bemerkungen gelten auch hier).

Beispiel 14

Glycerintricaprylat oder Tricaprylin (159; 2,8); Di-n-octadecyl-Zinn(IV)-Bis-(n-octadecylsulfonat), 4 %; $NH_3$-Mengen wie in Beispiel 4; Reaktionstemperaturen wie in Beispiel 4. Ergebnis: Glycerin-Ausbeute 89 %, Fettsäurenitril-Ausbeute 94 % mit 0,18 % Fettsäureamid und 0,12 % Fettsäure.

Beispiel 15

Glycerintristearat oder Tristearin (187; 6,7); DiphenylZinn(IV)-Bis-(n-octylsulfonat), 2 %; 600 1 $NH_3$ während der Glyceridumsetzung und 50 1 $NH_3$ während der Nachreaktion; Reaktionstemperaturen wie in Beispiel 3. Ergebnis: Glycerin-Ausbeute 92 %, Fettsäurenitril-Ausbeute 95 % mit 0,17 % Fettsäureamid und 0,14 % Fettsäure.

Beispiel 16

Glycerinmonolaurat (178; 3,8); Diphenyl-Zinn(IV)-Bis-(n-hexadecylsulfonat), 3 %; 800 1 $NH_3$ während der Glyceridumsetzung und 15 1 $NH_3$ während der Nachreaktion; Reaktionstemperaturen wie in Beispiel 6. Ergebnis: GlycerinAusbeute 92 %, Fettsäurenitril -Ausbeute 94 % mit 0,19 % Fettsäureamid und 0,12% Fettsäure.

Beispiel 17

Glycerinmonooleat (172; 4,6); Diphenyl-Zinn(IV)-Bis-(phenylsulfonat), 2 %; 400 1 $NH_3$ während der Glyceridumsetzung und 100 1 $NH_3$ während der Nachreaktion; Reaktionstemperaturen wie in Beispiel 1. Ergebnis: Glycerinausbeute 90 %, Fettsäurenitrilausbeute 90 % mit 0,14 % Fettsäureamid und 0,15 % Fettsäure.

Beispiel 18

Im Beispiel 18 wurden die nachstehend angegebenen Katalysatoren eingesetzt, wobei ansonsten jedesmal Beispiel 4 durchgeführt wurde. Die Katalysatoren sind: Zinkcaprylat, Zinkstearat, Zinkerucat, Blei-2-Methyl-benzoat, Zink-3-n-Dodecylbenzoat, Cadmium-2-Methylnaphthoat, Zink-Perfluorhexansulfonat, Kobalt-2-Hydroxy-n-dodecansulfonat und eine Mischung aus 1 Gew.-% Zink-n-Dodecylbenzolsulfonat und 1

Gew.-% Cadmium-Toluolsulfonat.
Ergebnis: Glycerin-Ausbeuten von 82 bis 96 Gew.-%, Fettsäurenitril-Ausbeuten von 89 bis 96 Gew.-% mit 0,15 bis 0,25 Gew.-% Fettsäureamid und 0,10 bis 0,20 Gew.-% Fettsäure.

## Ansprüche

1. Verfahren zur gleichzeitigen Herstellung von Fettsäurenitrilen und Glycerin aus Glyceriden, bei dem das Glycerid in einem Reaktionsgefäß bei einer Temperatur von 220 bis 300 °C mit Ammoniak in einer Menge von mindestens 200 Liter pro Kilogramm Glycerid pro Stunde in Gegenwart von für diese Umsetzung ausgewählten Katalysatoren umgesetzt und das bei der Glyceridumsetzung gebildete Produktgemisch aus dem Reaktionsgefäß ausgetragen wird, bis in dem ausgetragenen Produktgemisch, das im wesentlichen aus Wasser, Glycerin und Fettsäure und Fettsäureamid enthaltendem Fettsäurenitril besteht, praktisch kein Glycerin mehr enthalten ist, und bei dem anschließend das Fettsäure und Fettsäureamid enthaltende Fettsäurenitril in das Reaktionsgefäß zurückgebracht und in Gegenwart des obengenannten Katalysators bei einer Temperatur von 240 bis 320 °C mit Ammoniak in einer Menge von 5 bis 150 Liter pro Kilogramm im Reaktionsgefäß insgesamt vorliegendem Fettsäure und Fettsäureamid enthaltendem Fettsäurenitril pro Stunde weiter umgesetzt wird bis die gesamte Fettsäure und das gesamte Fettsäureamid in Fettsäurenitril umgewandelt sind, dadurch gekennzeichnet, daß das ausgetragene Fettsäure und Fettsäureamid enthaltende Fettsäurenitril anstelle der Rückführung nach der Glyceridumsetzung während der Glyceridumsetzung in die Reaktion zurückgeführt wird, wobei die oben angegebene Temperatur von 240 bis 320 °C und die oben angegebene Ammoniak-Menge von 5 bis 150 Liter dann eingestellt werden, sobald die Glyceridumsetzung praktisch beendet ist.

2. Verfahren nach Anspruch 1, dadurch gekennzeichnet, daß das Rückführen von Fettsäure und Fettsäureamid enthaltendem Fettsäurenitril kontinuierlich durchgeführt wird, indem die während der Glyceridumsetzung in der Zeiteinheit ausgetragene Menge im wesentlichen in der gleichen Zeiteinheit und im wesentlichen in der gleichen Menge in die Reaktion zurückgebracht wird.

3. Verfahren nach Anspruch 1 oder 2, bei dem das Produktgemisch aus dem Reaktionsgefäß über eine mit einer Kondensationseinrichtung verbundenen Leitung ausgetragen wird und bei dem die in der Kondensationseinrichtung kondensierten Produktkomponenten Glycerin und Fettsäure und Fettsäureamid enthaltendes Fettsäurenitril in einer beheizten Vorlage in eine untere Glycerin-Phase und in eine obere, Fettsäure und Fettsäureamid enthaltende Fettsäurenitril-Phase abgeschieden werden, dadurch gekennzeichnet, daß die Verbindungsleitung vom Reaktionsgefäß zur Kondensationseinrichtung während der Glyceridumsetzung im wesentlichen auf die Temperatur erhitzt wird, bei der die Glyceridumsetzung durchgeführt wird, und daß das in der Vorlage abgeschiedene Fettsäure und Fettsäureamid enthaltende Fettsäurenitril während der Glyceridumsetzung in die Reaktion zurückgeführt wird.

4. Verfahren nach Anspruch 3, dadurch gekennzeichnet, daß die Vorlage während der Glyceridumsetzung auf eine Temperatur von 140 bis 175 °C erhitzt wird.

5. Verfahren nach einem oder mehreren der Ansprüche 1 bis 4, dadurch gekennzeichnet, daß die Glyceridumsetzung bei einer Temperatur von 230 bis 270 °C durchgeführt wird, und daß nach Beendigung der Glyceridumsetzung eine Temperatur von 260 bis 300 °C und eine Ammoniak-Menge von 15 bis 100 Liter eingestellt und gehalten wird.

## Claims

1. A process for the simultaneous preparation of fatty acid nitriles and glycerol from glycerides, in which the glyceride is reacted, in a reaction vessel at a temperature of 220 to 300 °C, with ammonia in an amount of at least 200 liters per kilogram of glyceride per hour, in the presence of catalysts selected for this reaction, and the product mixture which is formed in the glyceride reaction is discharged from the reaction vessel until the discharged product mixture, which is essentially composed of water, glycerol, and fatty acid nitrile containing fatty acid and fatty acid amide, contains virtually no more glycerol, and in which thereafter the fatty acid nitrile containing fatty acid and fatty acid amide is returned to the reaction vessel and is further reacted, in the presence of the above-mentioned catalyst at a temperature of 240 to 320 °C, with ammonia in an amount of 5 to 150 liters per kilogram of the

total fatty acid nitrile containing fatty acid and fatty acid amide which is present in the reaction vessel, per hour until all the fatty acid and all the fatty acid amide have been converted into fatty acid nitrile, which comprises the discharged fatty acid nitrile containing fatty acid and fatty acid amide being returned to the reaction during the glyceride reaction, in place of return after the glyceride reaction, with the above-mentioned temperature of 240 to 320 °C, and the above-mentiond ammonia amount of 5 to 150 liters, being set up as soon as the glyceride reaction is virtually complete.

2. The process as claimed in claim 1, wherein the fatty acid nitrile containing fatty acid and fatty acid amide is continuously returned, in that the amount discharged per unit time during the glyceride reaction is returned to the reaction in essentially the same unit time and in essentially the same amount.

3. The process as claimed in claim 1 or 2, in which the product mixture is discharged from the reaction vessel via a line which is connected to a condensing device, and in which the product components glycerol and fatty acid nitrile containing fatty acid and fatty acid amide which have condensed in the condensing device are deposited in a heated receiver as a lower glycerol phase and an upper fatty acid nitrile phase containing fatty acid and fatty acid amide, comprises the connecting line from the reaction vessel to the condensing device being heated during the glyceride reaction essentially to the temperature which corresponds to the reaction temperature, and comprises the fatty acid nitrile which contains fatty acid and fatty acid amide and which is deposited in the receiver being returned to the reaction during the glyceride reaction.

4. The process as claimed in claim 3, wherein the receiver is heated during the glyceride reaction to a temperature of 140 to 175 °C.

5. The process as claimed in one or more of claims 1 to 4, wherein the glyceride reaction is carried out at a temperature of 230 to 270 °C, and wherein, after completion of the glyceride reaction, a temperature of 260 to 300 °C, and an amount of 15 to 100 liters of ammonia, is adjusted and maintained.

## Revendications

1. Procédé pour préparer simultanément des nitriles d'acide gras et du glycérol à partir de glycérides, selon lequel on fait réagir le glycéride, dans un réacteur à une température de 220 jusqu'à 300 °C, avec de l'ammoniac utilisé en une quantité d'au moins 200 l/kg de glycéride par heure, en présence de catalyseurs choisis pour cette réaction et l'on fait sortir du réacteur le mélange des produits formés lors de la réaction de transformation du glycéride jusqu'à ce que, dans le mélange des produits formés, consistant essentiellement en de l'eau, du glycérol et du nitrile d'acide gras contenant de l'acide gras et de l'amide d'acide gras, il n'y a pratiquement plus de glycérol, et selon lequel, ensuite, on fait revenir dans le réacteur le nitrile d'acide gras contenant l'acide gras et l'amide d'acide gras et, en présence du catalyseur précité, on fait encore réagir à une température de 240 à 320 °C avec de l'ammoniac utilisé en une quantité de 5 à 150 l/kg de nitrile d'acide gras présent en total dans le réacteur et contenant de l'acide gras et de l'amide d'acide gras, par heure, jusqu'à ce que la totalité de l'acide gras et la totalité de l'amide d'acide gras aient été transformées en nitrile d'acide gras, procédé caractérisé en ce que, au lieu d'effectuer un recyclage après la réaction de transformation du glycéride, on fait revenir dans le mélange réactionnel, pendant la réaction de transformation du glycéride, le nitirle d'acide gras, contenant de l'acide gras et de l'amide d'acide gras, qui étaient sortis du réacteur et l'on ajuste et maintient la température précitée de 240 à 320 °C et la quantité précitée d'ammoniac de 5 à 150 l dès que la réaction de transformation de glycéride est pratiquement achevée.

2. Procédé selon la revendication 1, caractérisé en ce qu'on conduit en continu le recyclage du nitrile d'acide gras contenant de l'acide gras et de l'amide d'acide gras, en réintroduisant dans le mélange réactionnel la quantité sortie dans un intervalle de temps pendant la réaction de transformation du glycéride, le recyclage s'effectuant essentiellement dans le même intervalle de temps et essentiellement en la même quantité.

3. Procédé selon la revendication 1 ou 2, caractérisé en ce que le mélange des produits est retiré ou entraîné du réacteur par l'intermédiaire d'un conduit relié à un dispositif de condensation et en ce qu'on soumet les prcduits composants, glycérol et nitrile d'acide gras contenant de l'acide gras et de

l'amide d'acide gras, condensés dans un dispositif de condensation et se trouvant dans un récipient récepteur chauffé, à une séparation en une phase inférieure de glycérol et en une phase supérieure de nitrile d'acide gras contenant de l'acide gras et de l'amide d'acide gras, procédé caractérisé en ce qu'on chauffe le conduit de liaison allant du réacteur au dispositif de condensation, pendant la réaction de transformation du glycéride, essentiellement à la température à laquelle la réaction de transformation de glycéride est conduite, et en ce qu'on recycle vers le mélange réactionnel, pendant la réaction de transformation du glycéride, le nitrile d'acide gras contenant l'acide gras et l'amide d'acide gras séparé dans le récipient récepteur.

**4.** Procédé selon la revendication 3, caractérisé en ce que, pendant la réaction de tranformation de glycéride, le récipient récepteur est chauffé à une température de 140 à 175 °c.

**5.** Procédé selon une ou plusieurs des revendications 1 à 4, caractérisé en ce qu'on conduit la réaction de transformation de glycéride à une température de 230 à 270 °C et en ce que, après achèvement de la réaction de transformation de glycéride, on règle et maintient à une température de 260 à 300 °C et à l'utilisation d'une quantité d'ammoniac de 15 à 100 l.